# EUROPEAN PATENT APPLICATION

(11) **EP 3 145 156 A1**
(43) Date of publication of application: **22.03.2017**
(21) Application number: 14896735.9
(22) Date of filing: 30.06.2014
(51) Int. Cl.: H04L 29/08

(54) **USER DATA PROCESSING METHOD AND DEVICE**

(71) Applicant: Huawei Technologies Co. Ltd., Shenzhen, Guangdong 518129 (CN)
(72) Inventor: GAN, Yuanli, Shenzhen Guangdong 518129 (CN); WANG, Hongjun, Shenzhen Guangdong 518129 (CN); SHAN, Zhenwei, Shenzhen Guangdong 518129 (CN); LIU, Bing, Shenzhen Guangdong 518129 (CN)
(74) Representative: Pfenning, Meinig & Partner mbB
(86) International application number: PCT/CN2014/081247
(87) International publication number: WO 2016/000163

(57) **Abstract**

Embodiments of the present invention provide a user data processing method, and a device. The method includes: acquiring, by a first device, first data and at least one piece of second data, wherein the first data is data acquired by the first device itself by detecting a user behavior, and the second data is data acquired by at least one second device by detecting the user behavior; and determining, by the first device in accordance with a preset rule according to the first data and the second data, user data corresponding to the user behavior, and presenting the user behavior and/or the user data, so that a user can view most correct and most needed data in the first time in different scenes, thereby improving user experience.

## Description

### TECHNICAL FIELD

Embodiments of the present invention relate to the field of communications technologies, and in particular, to a user data processing method, and a device.

### BACKGROUND

Nowadays, wearable smart devices gain popularity and there are increasingly more types of these devices. For example, there are smart watches, smart glasses, and smart bands. The foregoing devices are not only convenient to be carried, but also provide abundant utility functions for people. The appearance of the devices greatly changes life styles, exercise styles, and relaxation styles of modem people.

Currently, data such as step quantity, sleep, heart rate, and blood pressure is detected by using a sensor of a non-wearable smart device (for example, a mobile terminal such as a smartphone or a tablet computer) and is displayed on the non-wearable smart device. Alternatively, data such as step quantity, sleep, heart rate, and blood pressure is detected by using a sensor of a wearable smart device (for example, a smart band, a smart watch, or a smart ring) and is synchronously displayed on a non-wearable smart device.

With interconnection and intercommunication between devices, when data is transmitted and shared, and is displayed to a user in accordance with a specific rule, there are more detailed requirements. Currently, there is no good method for resolving a problem of how to enable a user to view most correct and most needed data in the first time in different scenes and a problem of how to record and display these data more scientifically when the user may carry both a wearable smart device and a non-wearable smart device.

### SUMMARY

Embodiments of the present invention provide a user data processing method, and a device, so that a user can view most correct and most needed data in the first time in different scenes, thereby improving user experience.

According to a first aspect, an embodiment of the present invention provides a user data processing method, including:
acquiring, by a first device, first data and at least one piece of second data, where the first data is data acquired by the first device itself by detecting a user behavior, and the second data is data acquired by at least one second device by detecting the user behavior; and
determining, by the first device in accordance with a preset rule according to the first data and the second data, user data corresponding to the user behavior, and presenting the user behavior and/or the user data.

In a first possible implementation manner of the first aspect, the preset rule includes:
dividing, within detection time from occurrence to stop of the user behavior, the detection time into multiple time segments in accordance with a preset time length; and for each time segment, selecting, according to a selection rule, one from the first data and the at least one piece of second data as user data corresponding to each time segment; and
summing the user data respectively corresponding to the time segments and using the sum as the user data of the user behavior.

With reference to the first possible implementation manner of the first aspect, in a second possible implementation manner, the method further includes:
the presenting the user behavior and/or the user data includes: displaying the user behavior and/or the user data in a form of coordinates, where the coordinates includes a time axis; and the displaying the user behavior and/or the user data in a form of coordinates specifically includes: if detecting, according to the first data and the at least one piece of second data, that a moving track of the user behavior is located in a same region within a detection time segment, calculating a center point of the region, and displaying, at a point corresponding to the detection time segment on a time axis on a display screen of the first device, the user behavior occurring at the center point.

In a third possible implementation manner of the first aspect, the method further includes:
obtaining, by the first device, a status of the user behavior by determining according to the first data and the at least one piece of second data, where
correspondingly, the preset rule includes: selecting, according to the status of the user behavior, the first data having a high priority or the second data having a high priority as the user data corresponding to the user behavior.

According to a second aspect, an embodiment of the present invention provides a first device, including:
an acquiring module, configured to acquire first data and at least one piece of second data, where the first data is data acquired by the first device itself by detecting a user behavior, and the second data is data acquired by at least one second device by detecting the user behavior;
a processing module, configured to determine, by the first device in accordance with a preset rule according to the first data and the second data, user data corresponding to the user behavior; and
a presentation module, configured to present the user behavior and/or the user data.

In a first possible implementation manner of the second aspect, the preset rule includes:
dividing, within detection time from occurrence to stop of the user behavior, the detection time into multiple time segments in accordance with a preset time length; and for each time segment, selecting, according to a selection rule, one from the first data and the at least one piece of second data as user data corresponding to each time segment; and
summing the user data respectively corresponding to the time segments and using the sum as the user data of the user behavior.

With reference to the first possible implementation manner of the second aspect, in a second possible implementation manner, the preset rule further includes: if detecting, according to the first data and the at least one piece of second data, that a moving track of the user behavior is located in a same region within a detection time segment, calculating a center point of the region; and
correspondingly, the presentation module is further configured to display, at a point corresponding to the detection time segment on a time axis on a display screen of the first device, the user behavior occurring at the center point.

In a third possible implementation manner of the second aspect, the device further includes:
a determining module, configured to obtain, by the first device, a status of the user behavior by determining according to the first data and the at least one piece of second data, where
correspondingly, the preset rule includes: selecting, according to the status of the user behavior, the first data having a high priority or the second data having a high priority as the user data corresponding to the user behavior.

In the user data processing method, and the device that are provided in the embodiments of the present invention, a first device acquires first data that is acquired by the first device itself by detecting a user behavior, and at least one piece of second data that is acquired by a second device by detecting the user behavior, and then, determines, in accordance with a preset rule, user data corresponding to the user behavior, and presents the user behavior and/or the user data on the first device, so that a user can view most correct and most needed data in the first time in different scenes, thereby improving user experience.

### BRIEF DESCRIPTION OF DRAWINGS

To describe the technical solutions in the embodiments of the present invention or in the prior art more clearly, the following briefly describes the accompanying drawings required for describing the embodiments or the prior art. Apparently, the accompanying drawings in the following description show some embodiments of the present invention, and a person of ordinary skill in the art may still derive other drawings from these accompanying drawings without creative efforts.
FIG. 1 is a flowchart of Embodiment 1 of a user data processing method according to the present invention;
FIG. 2 is a flowchart of Embodiment 2 of a user data processing method according to the present invention;
FIG. 3 is a flowchart of Embodiment 3 of a user data processing method according to the present invention;
FIG. 4 is a schematic structural diagram of Embodiment 1 of a first device according to the present invention; and
FIG. 5 is a schematic structural diagram of Embodiment 2 of the first device according to the present invention.

### DESCRIPTION OF EMBODIMENTS

To make the objectives, technical solutions, and advantages of the embodiments of the present invention clearer, the following clearly and completely describes the technical solutions in the embodiments of the present invention with reference to the accompanying drawings in the embodiments of the present invention. Apparently, the described embodiments are some but not all of the embodiments of the present invention. All other embodiments obtained by a person of ordinary skill in the art based on the embodiments of the present invention without creative efforts shall fall within the protection scope of the present invention.

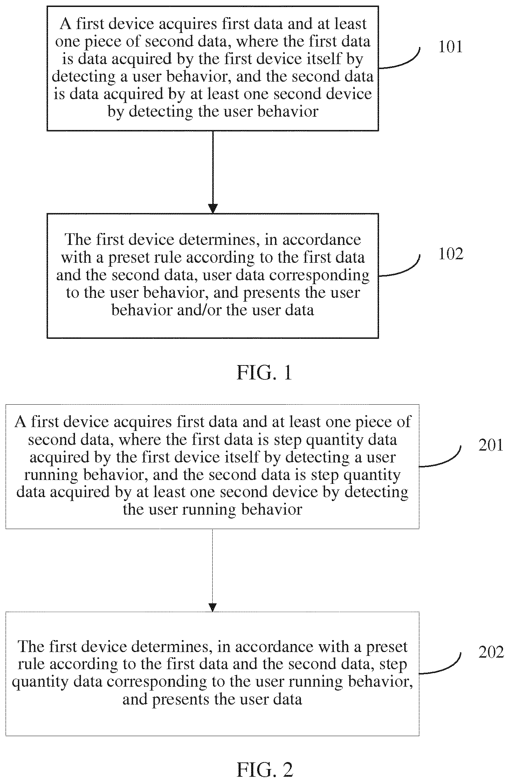

FIG. 1 is a flowchart of Embodiment 1 of a user data processing method according to the present invention. As shown in FIG. 1, the method of this embodiment may include:
Step 101: A first device acquires first data and at least one piece of second data, where the first data is data acquired by the first device itself by detecting a user behavior, and the second data is data acquired by at least one second device by detecting the user behavior.

The first device may be a non-wearable smart device or a wearable smart device, and the second device is a wearable smart device. For example, the non-wearable smart device may be a smartphone, a tablet computer, or the like; and the wearable smart device may be a pair of smart glasses, a smart watch, a smart ring, or the like.

The first device acquires the first data and the at least one piece of second data, which may be specifically understood as that: the first device acquires the data that is acquired by the first device itself by detecting the user behavior and the data that is acquired by the second device by detecting the user behavior, that is, the first data is the data acquired by the first device itself, and the second data is the data acquired by the second device itself.

Step 102: The first device determines, in accordance with a preset rule according to the first data and the second data, user data corresponding to the user behavior, and presents the user behavior and/or the user data.

The first device determines, in accordance with the preset rule according to the acquired first data and the acquired second data, the user data corresponding to the user behavior, and presents the user behavior and/or the user data on the first device, where the user data is data determined from the acquired first data and the acquired second data according to the preset rule. In addition, the presentation is not limited to visual sense, and may also include auditory sense, tactile sense, taste sense, or the like.

In this embodiment, a first device acquires first data that is acquired by the first device itself by detecting a user behavior, and at least one piece of second data that is acquired by a second device by detecting the user behavior, and then, determines, in accordance with a preset rule, user data corresponding to the user behavior, and presents the user behavior and/or the user data on the first device, so that a user can view most correct and most needed data in the first time in different scenes, thereby improving user experience.

The preset rule in the foregoing embodiment may include: dividing, within detection time from occurrence to stop of the user behavior, the detection time into at least one time segment in accordance with a preset time length; and for each time segment, selecting, according to a selection rule, one from the first data and the at least one piece of second data as user data corresponding to the time segment. The following gives detailed description with reference to an embodiment of FIG. 2.

FIG. 2 is a flowchart of Embodiment 2 of a user data processing method according to the present invention. As shown in FIG. 2, the method of this embodiment may include:
Step 201: A first device acquires first data and at least one piece of second data, where the first data is step quantity data acquired by the first device itself by detecting a user running behavior, and the second data is step quantity data acquired by at least one second device by detecting the user running behavior.

In this embodiment, the first device is, for example, a smartphone, and the second device is, for example, a smart band and a pair of smart shoes. This embodiment is described by using an example in which running is used as a user behavior. The first data is the step quantity data acquired by the smartphone itself by detecting the user running behavior, and the second data is the step quantity data acquired by the smart band and/or the pair of smart shoes by detecting the user running behavior.

Step 202: The first device determines, in accordance with a preset rule according to the first data and the second data, step quantity data corresponding to the user running behavior, and presents the user data.

Specifically, within detection time from occurrence to stop of the user running behavior, if the smartphone has step quantity data, and the smart band or the pair of smart shoes does not have step quantity data, the step quantity data acquired by the smartphone is selected. If the smartphone does not have step quantity data, the smart band has step quantity data, and the pair of smart shoes does not have step quantity data, the step quantity data acquired by the smart band is selected. If the smartphone does not have step quantity data, the smart band does not have step quantity data either, and the pair of smart shoes has step quantity data, the step quantity data acquired by the pair of smart shoes is selected, that is, the acquired step quantity data is selected as user data.

Within the detection time from occurrence to stop of the user running behavior, at least two of the smartphone, the smart band, and the pair of smart shoes have step quantity data. In this case, data record points are subdivided, where in this embodiment, every five minutes is one point. For example, one hour is divided into 12 segments of time. Within each time segment, step quantity data with a relatively large amount of exercise is selected as the step quantity data. For example, if within the first five-minute time segment, the step quantity acquired by the smartphone is 150, the step quantity acquired by the smart band is 158, and the step quantity acquired by the pair of smart shoes is 160, data of the step quantity that is acquired by the pair of smart shoes and is 160 is selected as step quantity data in the first five-minute time segment. The step quantity data in the other time segments may be deduced by analogy. Certainly, the time of five minutes is not absolute, but relative, and the specific time may be determined by capabilities of a smart device, which is not limited herein.

Then, the step quantity data respectively corresponding to the time segments is summed up to obtain final user data within the detection time from occurrence to stop of the user running behavior, and the final user data is presented on the smartphone.

In this embodiment, a smartphone acquires step quantity data that is acquired by the smartphone itself by detecting a user running behavior and step quantity data that is acquired by a smart band and a pair of smart shoes by detecting the user running behavior; then, subdivides data record points, and within each time segment, selects data with a relatively large amount of exercise as the user data. Finally, sums the user data respectively corresponding to time segments to obtain final user data within detection time from occurrence to stop of the user running behavior, and presents the final user data on the smartphone, so that a user can view most correct and most needed data in the first time in different scenes, thereby improving user experience.

Based on the foregoing embodiment, in this method embodiment, positions of a user within certain time may also be displayed on a corresponding time axis according to the user data obtained by the terminal device. In this embodiment, the first data may be longitude and latitude obtained by the first device (such as a smartphone), and the second data may be longitude and latitude obtained by the second device (such as a smart watch). This embodiment is described by using an example in which a longitude and latitude point is recorded every 30 seconds, and a specific time interval may be configured according to an actual status, which is not limited herein.

When the smartphone acquires longitude and latitude data, and the smart watch does not acquire longitude and latitude data, the longitude and latitude data acquired by the smartphone is used; or when the smartphone does not acquire longitude and latitude data, and the smart watch acquires longitude and latitude data, the longitude and latitude data acquired by the smart watch is used.

When both the smartphone and the smart watch acquire the longitude and latitude data, at a point at which time segments overlap, the longitude and latitude data acquired by the smart watch is used because the longitude and latitude data of the smart watch is from a GPS, while the longitude and latitude data of the smartphone may be from the GPS, or from a base station or WIFI and the longitude and latitude data provided by the base station or the WIFI is not precise and has a deviation.

The presenting the user behavior and/or the user data includes: displaying the user behavior and/or the user data in a form of coordinates, where the coordinates includes a time axis; and the displaying the user behavior and/or the user data in a form of coordinates specifically includes: if detecting, according to the first data and the at least one piece of second data, that a moving track of the user behavior is located in a same region within a detection time segment, calculating a center point of the region, and displaying, at a point corresponding to the detection time segment on a time axis on a display screen of the first device, the user behavior occurring at the center point.

The smartphone draws the moving track of the user according to the acquired longitude and latitude data, obtains through aggregation a range of activities of the user within the time segment. If detecting that the moving track of the user behavior is located in a same region within a detection time segment, the smartphone calculates a center point of the region, and displays, on the time axis on the display screen of the smartphone, the user behavior that occurs at the center point within the detection time segment.

In this embodiment, a non-wearable smart device such as a smartphone acquires longitude and latitude data that is acquired by the non-wearable smart device itself by detecting a user behavior, and longitude and latitude data that is acquired by a wearable smart device such as a smart watch by detecting the user behavior, and then, draws according to the acquired longitude and latitude points, to obtain a moving track of a user, and obtains through aggregation a range of activities of the user within a time segment. If it is detected that the moving track of the user behavior is located in a same region within a detection time segment, a center point of the same region in which the moving track of the user behavior is located is calculated, and the user behavior occurring at the center point within the detection time segment is displayed on a time axis on a display screen of the smartphone, so that the user views most correct and most needed data in the first time in different scenes, thereby improving user experience.

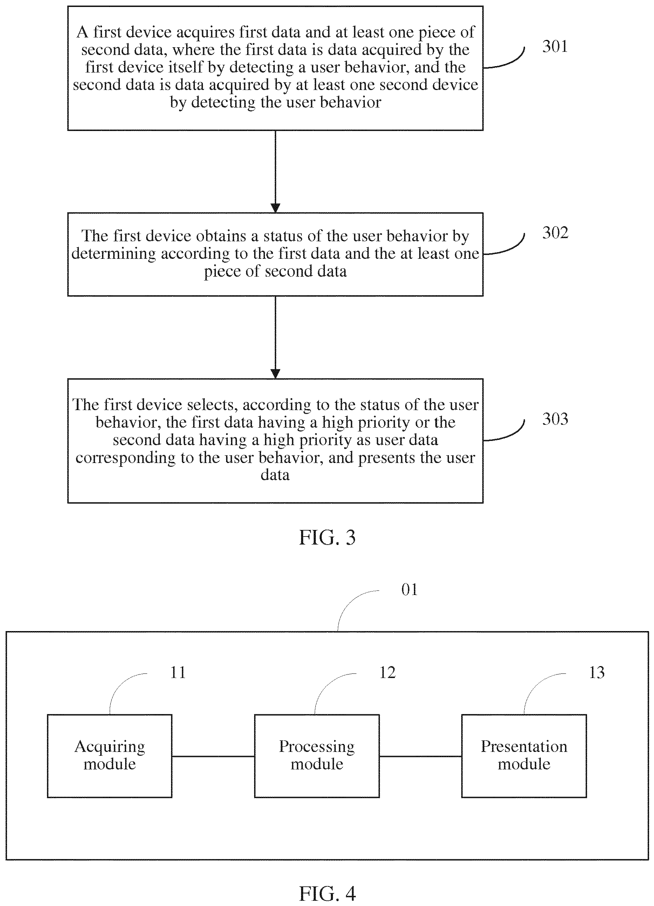

The preset rule described in Embodiment 1 may further include: selecting, according to a status of the user behavior, the first data having a high priority or the second data having a high priority as the user data corresponding to the user behavior. The following gives detailed description with reference to an embodiment of FIG. 3.

FIG. 3 is a flowchart of Embodiment 3 of a user data processing method according to the present invention. As shown in FIG. 3, the method of this embodiment may include:
Step 301: A first device acquires first data and at least one piece of second data, where the first data is data acquired by the first device itself by detecting a user behavior, and the second data is data acquired by at least one second device by detecting the user behavior.

In this embodiment, description is given by using an example in which the first device is a smartphone, a pair of smart glasses, and a smart watch, and the second device is a pair of smart glasses, a smart watch, a pair of smart shoes, a smart band, and a smart ring.

The first data is the data acquired by the first device itself by detecting the user behavior, that is, the data acquired by the smartphone, the pair of smart glasses, and the smart watch by detecting the user behavior, for example, data such as step quantity, heart rate, and blood pressure. The second data is the data acquired by the at least one second device by detecting the user behavior, that is, the data acquired by the pair of smart shoes, the smart band, and the smart ring by detecting the user behavior, for example, data such as step quantity, heart rate, and blood pressure.

First, the pair of smart shoes, the smart band, and the smart ring periodically send broadcast ADV_IND packets; after receiving the ADV_IND packets, the smartphone, the pair of smart glasses, and the smart watch broadcast SCAN_REQ packets and scan nearby Bluetooth devices; after receiving the SCAN_REQ packet, the pair of smart shoes, the smart band, and the smart ring respond with SCAN_RSP packets, where the SCAN_RSP packets carry information such as identity numbers (IDentity, ID for short) of the devices and Bluetooth addresses of the devices; after receiving the SCAN_RSP packets, the smartphone, the pair of smart glasses, and the smart watch establish, according to the Bluetooth addresses of the devices, connections to corresponding devices, and acquire capabilities of devices such as the pair of shoes, the smart band, and the smart ring, for example, information about services supported by the devices.

Then, the smart devices such as the smartphone, the pair of smart glasses, and the smart watch acquire data that is acquired by the smart devices themselves by detecting the user behavior, and data that is acquired by the smart devices such as the pair of smart shoes, the smart band, and the smart ring by detecting the user behavior.

Step 302: The first device obtains a status of the user behavior by determining according to the first data and the at least one piece of second data.

Motion sensors (such as an acceleration sensor, a gravity sensor, and a gyroscope) on the smartphone, the pair of smart glasses, and the smart watch are used to identify a status of a user; or, the status of the user behavior is identified by the devices such as the smartphone, the pair of smart glasses, and the smart watch by collecting and integrating data acquired by the devices themselves or data acquired from the pair of smart shoes, the smart band, and the smart ring, where the status of the user behavior is, for example, motion, standstill, or sleep.

Step 303: The first device selects, according to the status of the user behavior, the first data having a high priority or the second data having a high priority as the user data corresponding to the user behavior, and presents the user data.

Specifically, a user data priority policy is configured on a first device (this embodiment gives description by using only a smartphone, a pair of smart glasses, and a smart watch as an example) having a processor; when the smartphone, the pair of smart glasses, and the smart watch devices are all worn on a user, the user data priority policy configured on the smartphone ranks the first, the user data priority policy configured on the pair of smart glasses ranks the second, and the user data priority policy configured on the smart watch ranks the third. In this embodiment, description is given by using only step quantity data, heart rate data, blood pressure data, and sleep quality data as an example.

The following is detailed content of the priority policy:

When a user is in a moving status and step quantity data needs to be acquired, step quantity data acquired by a pair of smart shoes or a smart foot band is selected preferentially; if data of a sensor on the pair of smart shoes or the smart foot band cannot be obtained, step quantity data acquired by a smart band or a smart watch is selected, and next, step quantity data acquired by a smart ring or a pair of smart glasses is selected, that is, the priority sequence is that the pair of smart shoes or the smart foot band > the smart band or the smart watch > the smart ring or the pair of smart glasses; and
when heart rate or blood pressure data needs to be acquired, heart rate or blood pressure data acquired from the smart band or the smart watch is selected preferentially; if data of a sensor on the smart band or the smart watch cannot be obtained, heart rate or blood pressure data acquired by the smart ring is selected, and next, heart rate or blood pressure data acquired by the smart foot band or the pair of smart shoes is selected, that is, the priority sequence is that the smart band or the smart watch > the smart ring > the smart foot band or the pair of smart shoes.

When a user is in a sleeping status and sleep quality data (the sleep quality is often implemented by using data such as dreams, pulses, and body motion records) needs to be acquired, sleep quality data acquired from the smart band or the smart watch is preferentially selected. If data of a sensor on the smart band or the smart watch cannot be obtained, sleep quality data acquired by the smart ring is selected, and next, heart rate or blood pressure data acquired by the smart foot band is selected, that is, the priority sequence is that the smart band or the smart watch > the smart ring > the smart foot band.

A priority policy is configured according to behavior habits of a user; for example, in most cases, the user preferentially acquires the step quantity data from the pair of smart shoes or the smart foot band by default; then, acquires the step quantity data from the smart band or the smart watch; and then, acquires the step quantity data from the smart ring or the pair of smart glasses. However, when a user has personalized settings, for example, the user first acquires the step quantity data from the smart ring or the pair of smart glasses; then, acquires the step quantity data from the pair of smart shoes or the smart foot band; and then, acquires the step quantity data from the smart band or the smart watch. The priority policy is configured in accordance with personalized requirements of the user; that is, the current priority sequence is that: the smart ring or the pair of smart glasses > the pair of smart shoes or the smart foot band > the smart band or the smart watch.

Then, the corresponding user data selected by using the foregoing priority policy is presented on the smartphone, the pair of smart glasses, the smart watch, the pair of smart shoes, the smart band, and the smart ring, where the presentation manner may be multiple manners such as visual sense, auditory sense, tactile sense, and taste sense. For example, the corresponding user data is displayed on the smartphone, the pair of smart glasses, and the smart watch; the corresponding user data is played by the smartphone in an audio manner; and the corresponding user data is prompt to the user by the pair of smart shoes, the smart band, and the smart ring in a vibration manner.

In this embodiment, smart devices such as a smartphone, a pair of smart glasses, and a smart watch acquire data that is acquired by the smart devices themselves by detecting a user behavior, and data that is acquired by smart devices such as a pair of smart shoes, a smart band, and a smart ring by detecting the user behavior. Then, a status of the user behavior is identified according to the acquired data. Subsequently, user data priority policies are configured on devices having a processor, such as a smartphone, a pair of smart glasses, and a smart watch, and the corresponding user data selected by using the priority policy is presented on the smartphone, the pair of smart glasses, the smart watch, the pair of smart shoes, the smart band, and the smart ring, so that a user can view most correct and most needed data in the first time in different scenes, thereby improving user experience.

FIG. 4 is a schematic structural diagram of Embodiment 1 of a first device according to the present invention. As shown in FIG. 4, the first device 01 of this embodiment may include: an acquiring module 11, a processing module 12, and a presentation module 13, where the acquiring module 11 is configured to acquire first data and at least one piece of second data, where the first data is data acquired by the first device itself by detecting a user behavior, and the second data is data acquired by at least one second device by detecting the user behavior; the processing module 12 is configured to determine, by the first device in accordance with a preset rule according to the first data and the second data, user data corresponding to the user behavior; and the presentation module 13 is configured to present the user behavior and/or the user data.

Based on the foregoing embodiment, specifically, the preset rule may include: dividing, within detection time from occurrence to stop of the user behavior, the detection time into multiple time segments in accordance with a preset time length; for each time segment, selecting, according to a selection rule, one from the first data and the at least one piece of second data as user data corresponding to the time segment; and summing the user data respectively corresponding to the time segments and using the sum as the user data of the user behavior.

Further, the preset rule may include: if detecting, according to the first data and the at least one piece of second data, that a moving track of the user behavior is located in a same region within a detection time segment, calculating a center point of the region; and
correspondingly, the presentation module 13 is further configured to display, at a point corresponding to the detection time segment on a time axis on a display screen of the first device, the user behavior occurring at the center point.

The first device in this embodiment may be configured to execute the technical solutions of the foregoing method embodiments. The implementation principle and technical effect of the first device are similar to those of the method, and no further details are described herein again.

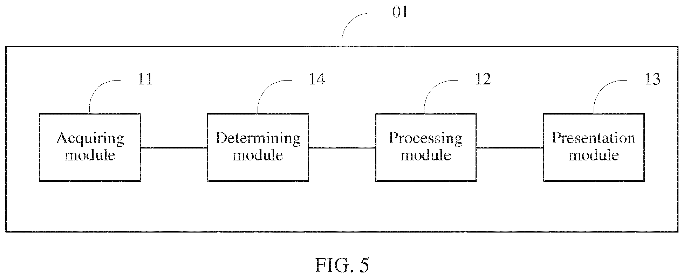

FIG. 5 is a schematic structural diagram of Embodiment 2 of the first device according to the present invention. As shown in FIG. 4, based on the structure of the device shown in FIG. 4, the first device 01 of this embodiment may further include: a determining module 14, where the determining module 14 is configured to obtain, by the first device, a status of the user behavior by determining according to the first data and the at least one piece of second data.

Correspondingly, the preset rule may include: selecting, according to the status of the user behavior, the first data having a high priority or the second data having a high priority as the user data corresponding to the user behavior.

The first device in this embodiment may be configured to execute the technical solutions of the foregoing method embodiments. The implementation principle and technical effect of the first device are similar to those of the method, and no further details are described herein again.

In the several embodiments provided in the present invention, it should be understood that the disclosed device and method may be implemented in other manners. For example, the described device embodiment is merely exemplary. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the devices or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected according to actual needs to achieve the objectives of the solutions of the embodiments.

In addition, functional units in the embodiments of the present invention may be integrated into one processing unit, or each of the units may exist alone physically, or two or more units are integrated into one unit. The integrated unit may be implemented in a form of hardware, or may be implemented in a form of hardware in addition to a software functional unit.

When the foregoing integrated unit is implemented in a form of a software functional unit, the integrated unit may be stored in a computer-readable storage medium. The software functional unit is stored in a storage medium and includes several instructions for instructing a computer device (which may be a personal computer, a server, or a network device) or a processor to perform a part of the steps of the methods described in the embodiments of the present invention. The foregoing storage medium includes: any medium that can store program code, such as a USB flash drive, a removable hard disk, a read-only memory (Read-Only Memory, ROM), a random access memory (Random Access Memory, RAM), a magnetic disk, or an optical disc.

It may be clearly understood by a person skilled in the art that, for the purpose of convenient and brief description, division of the foregoing function modules is taken as an example for illustration. In actual application, the foregoing functions can be allocated to different function modules and implemented according to a requirement, that is, an inner structure of an apparatus is divided into different function modules to implement all or part of the functions described above. For a detailed working process of the foregoing device, reference may be made to a corresponding process in the foregoing method embodiments, and details are not described herein again.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions of the embodiments of the present invention.

## Claims

1. A user data processing method, comprising:
acquiring, by a first device, first data and at least one piece of second data, wherein the first data is data acquired by the first device itself by detecting a user behavior, and the second data is data acquired by at least one second device by detecting the user behavior; and
determining, by the first device in accordance with a preset rule according to the first data and the second data, user data corresponding to the user behavior, and presenting the user behavior and/or the user data.

2. The method according to claim 1, wherein the preset rule comprises:
dividing, within detection time from occurrence to stop of the user behavior, the detection time into multiple time segments in accordance with a preset time length; and for each time segment, selecting, according to a selection rule, one from the first data and the at least one piece of second data as user data corresponding to each time segment; and
summing the user data respectively corresponding to the time segments and using the sum as the user data of the user behavior.

3. The method according to claim 2, wherein the presenting the user behavior and/or the user data comprises: displaying the user behavior and/or the user data in a form of coordinates, wherein the coordinates comprises a time axis; and the displaying the user behavior and/or the user data in a form of coordinates specifically comprises:
if detecting, according to the first data and the at least one piece of second data, that a moving track of the user behavior is located in a same region within a detection time segment, calculating a center point of the region, and displaying, at a point corresponding to the detection time segment on the time axis on a display screen of the first device, the user behavior occurring at the center point.

4. The method according to claim 1, wherein the method further comprises:
obtaining, by the first device, a status of the user behavior by determining according to the first data and the at least one piece of second data, wherein
correspondingly, the preset rule comprises: selecting, according to the status of the user behavior, the first data having a high priority or the second data having a high priority as the user data corresponding to the user behavior.

5. A first device, comprising:
an acquiring module, configured to acquire first data and at least one piece of second data, wherein the first data is data acquired by the first device itself by detecting a user behavior, and the second data is data acquired by at least one second device by detecting the user behavior;
a processing module, configured to determine, by the first device in accordance with a preset rule according to the first data and the second data, user data corresponding to the user behavior; and
a presentation module, configured to present the user behavior and/or the user data.

6. The first device according to claim 5, wherein the preset rule comprises:
dividing, within detection time from occurrence to stop of the user behavior, the detection time into multiple time segments in accordance with a preset time length; and for each time segment, selecting, according to a selection rule, one from the first data and the at least one piece of second data as user data corresponding to each time segment; and
summing the user data respectively corresponding to the time segments and using the sum as the user data of the user behavior.

7. The first device according to claim 6, wherein the presenting the user behavior and/or the user data comprises: displaying the user behavior and/or the user data in a form of coordinates, wherein the coordinates comprises a time axis; and the displaying the user behavior and/or the user data in a form of coordinates specifically comprises: if detecting, according to the first data and the at least one piece of second data, that a moving track of the user behavior is located in a same region within a detection time segment, calculating a center point of the region; and
correspondingly, the presentation module is further configured to display, at a point corresponding to the detection time segment on a time axis on a display screen of the first device, the user behavior occurring at the center point.

8. The first device according to claim 5, wherein the device further comprises:
a determining module, configured to obtain, by the first device, a status of the user behavior by determining according to the first data and the at least one piece of second data, wherein
correspondingly, the preset rule comprises: selecting, according to the status of the user behavior, the first data having a high priority or the second data having a high priority as the user data corresponding to the user behavior.
